# EUROPEAN PATENT SPECIFICATION

(11) **EP 1 929 962 B1**
(45) Date of publication and mention of the grant of the patent: **16.12.2009**
(21) Application number: 05753808.4
(22) Date of filing: 27.05.2005
(51) Int. Cl.: A61B 17/062

(54) **SURGICAL NEEDLE HOLDER FOR STITCH SUTURING**
HALTER FÜR CHIRURGISCHE NADEL ZUM NÄHEN
PORTE-AIGUILLES CHIRURGICAL PERMETTANT DE FAIRE DES SUTURES

(43) Date of publication of application: 11.06.2008
(73) Proprietor: Instituto Tecnologico de Canarias, S.A. (ITC), 35119 Santa Lucia, Las Palmas (ES); Diaz de Tuesta Revilla, Ignacio, 35119 Santa Lucia Las Palmas (ES)
(72) Inventor: MONOPOLI FORLEO, Donato, E-35119 Las Palmas de Gran Canaria (ES); MENTADO ALMEIDA, Belinda, E-35119 Las Palmas de Gran Canaria (ES); DIAZ DE TUESTA REVILLA, Ignacio, E-35119 Las Palmas de Gran Canaria (ES)
(74) Representative: Gomez-Acebo, Ignacio
(86) International application number: PCT/ES2005/000302
(87) International publication number: WO 2006/125835

(56) References cited:
- EP-A1- 1 498 075
- EP-A2- 1 250 891
- US-A- 3 168 097
- US-A- 5 797 956
- US-A1- 2002 055 758

## Description

### OBJECT OF THE INVENTION

The present invention relates to a surgical needle holder for stitch suturing, specifically to facilitate suturing in areas or parts that are difficult to access by the surgeon's hand or areas that require suturing in which manual manipulation of the needle is not possible, or in which it is more convenient to manipulate by means of a device.

The object of the invention is to provide a needle-holding device that allows access to remote areas, in addition to enabling high-precision suturing, even in those cases in which the plane along which the needle is displaced coincides with the longitudinal axle of the needle holder, or remains close to it. That is, it allows movement of the needle along the aforementioned planes without having to perform complex arm movements to transmit the rotation movement.

### BACKGROUND OF THE INVENTION

Occasionally, suturing to stitch human body tissues together cannot be performed manually as a consequence of the difficulties encountered by the surgeon's hand or because the needle requires a firmer grip.

The existence of devices and strategies that solve this problem and make these practices feasible is known, said devices being called needle holders which, in medical jargon, are known as "holders", which serve to manipulate needles remotely.

Consequently, the function of the needle holders is that of extending the action of the surgeon's hand, allowing access to parts of the human body that could otherwise not be accessed by a hand, while increasing the strength of the hand so as to exert sufficient pressure on the needle to avoid it from slipping as it penetrates the tissues.

However, the structural characteristics and complexity of these devices pose several inconveniences.

Amongst said problems we must firstly highlight limited access to areas that require suturing, due to the characteristics of the interventions themselves, which normally produce the smallest possible wounds to access the intervention areas.

Another problem is the orientation of the suture plane with respect to the instrument, as existing needle holders lose effectiveness when the needle plane approaches that of the device or, strictly speaking, needle holder.

Suturing is normally performed in such a manner that the needle plane is perpendicular to the longitudinal plane of the holder, so that once the holder is gripped, the rotating movement of the forearm (pronosupination) displaces the needle along its movement plane. But if the needle must be moved along a plane that is not perpendicular to the longitudinal axle of the holder, the degree of difficulty and imprecision increases, as the holder must not only rotate around its axle but also describe a translational movement relative to said axle, which on occasions is not possible due to the limited space provided by access wounds.

The two aforementioned problems are very common in interventions such as implantation of heart valves, mini-invasive surgery, transplants and in general interventions performed through limited spaces. Exclusively mechanical devices aimed at moving the needle in a natural manner along planes that include the longitudinal axle of the holder have not yet been devised.

All of the holders designed to date grip the needle along a plane perpendicular to its axle, and are aimed at ensuring that the forearm's rotation along its "longitudinal axle" is transmitted to the longitudinal axle of the holder in order to rotate the needle along said plane. At present, only electromechanical devices (surgical robots, which are really telemanipulators) allow the needle to move along planes perpendicular to the arm's axle by means of two universal articulations which are rotated by means of pulleys and tensioners that actuate motors. A needle holder according to the preamble of claim 1 is known from the document US-2002/0055758.

### DESCRIPTION OF THE INVENTION

The surgical needle holder of the invention has been conceived to solve the aforementioned problems, as it is configured in such a manner as to manipulate and move needles along any plane parallel to the longitudinal axle of the device.

More specifically, the needle holder is comprised of two arms, namely a mobile arm and fixed arm, the latter being longer than the former, with the peculiarity that both arms end in expansions in the form of scissor grip eyes for introducing the fingers and correspondingly actuating said mobile arm.

The fixed arm extends, from the end of the mobile arm, along a segment of considerable length, on the free end of which a head is mounted which enables two possible movements, a rotating movement to perform the necessary movements while suturing, and an opening and closing movement to release or adequately grip the needle.

The device or needle holder assembly includes means by which to carry out the movements of the aforementioned head.

In a first embodiment of the surgical needle holder, the head is comprised of a fixed and mobile part, which opens and closes to release or grip the needle, both parts forming a clamp which is mounted on the same axle of a pulley by means of which the head rotates.

In this variant of embodiment, the actuation of the two parts that comprise the actual clamp or head, to open or close it, is carried out by actuating a pawl mounted on the fixed arm. A cable is disposed between the pawl and the mobile part of said clamp or head which, in a determined position, is tensed, thereby pressing the mobile part of the clamp against the fixed part and therefore gripping the suture needle which in this type of instruments are known to be curved.

The aforementioned actuating pawl is related to a ratchet on which said pawl is locked in any actuation position, said ratchet being complementarily pushed towards a blocking position by a spring, so that the manual actuation of the ratchet, in the manner of a second pawl, counter to the spring action, releases the ratchet from the pawl, thereby allowing detensioning of the cable so as to release the needle gripped by the clamp, as in this case the mobile part shall be displaced laterally outwards, becoming separated from the other fixed part and releasing the suture needle.

In this variant of embodiment, the rotating actuation of the aforementioned head or clamp and therefore movement towards the right and left of the needle to enable its function, is carried out by means of a rotating movement that is transmitted by a belt to the pulley related to the aforementioned head, said belt being related by its other end to a second pulley which is considered the driving pulley, disposed at the side of a mechanism box essentially comprised of two geared toothed wheels that rotate in opposite directions, i.e. when one rotates in a clockwise direction the other rotates in an anti-clockwise direction and vice versa, said mechanism being actuated by the tilting of the mobile arm, which is actuated in a scissor-like manner, the rotation direction being determined by means of an external mobile selector related to said mechanism box, in such a manner that a pinion is in turn geared to either of said wheels, susceptible to displacement about its own axle for gearing to either of the wheels, thereby achieving clockwise or anti-clockwise rotation in accordance with the toothed wheel selected, with the peculiarity that the pulley considered the driving pulley is external and laterally mounted on the mounting axle of the pinion geared to the two toothed wheels.

In this manner, the rotating movement of the driving pulley is achieved and therefore that of the pulley mounted on the head, enabling the latter to rotate in a clockwise or anti-clockwise direction, accordingly, and allowing the surgeon to adequately perform the suturing with the needle gripped by the head or clamp of which it is comprised.

Transmission of movement may also be rigid, using axles or gears, in substitution of the aforementioned pulleys and cable or belt.

In a variant of embodiment, the head is comprised of a toothed wheel that comprises a body through which an axle that comes into contact with a mobile part that forms part of the head passes transversely, and logically through the wheel itself, in such a manner that said head may tilt towards opening and closing positions with respect to the fixed part of the body that also forms part of the toothed wheel, so as to release or grip the suture needle accordingly.

The aforementioned head and toothed wheel assembly is mounted on the free end of the longer arm, in such a manner that the toothed wheel is disposed within a cavity of said arm, while the rest of the head body projects towards one of the sides, whereas the end of the pass-through axle projects towards the other side through the toothed wheel of the lateral body thereof, wherein a lateral part or plate comes into contact with the projecting end of the axle, the tilting movement of which can occupy two positions, one in which it pushes against the aforementioned end of the axle, therefore forcing it to project towards the other side, of the head itself, and come into contact with the mobile part thereof, thereby opening it; and another resting position in which the lateral plate does not come into contact with the aforementioned axle and therefore maintains the head in its open position.

The tilting movement of this lateral plate is actuated by an extension disposed at the end of the shorter arm articulation, which is mounted on an axle around which it may rotate so that said extension to the shorter arm comes into contact with the fore-end of the lateral plate, thereby producing the tilting movement thereof so that it may come into contact, by its other end, with the aforementioned closing axle of the head, having foreseen the downward extension of the aforementioned rotation axle of the shorter arm to form a type of fork, the branches of which are disposed in holes made for said purpose in a movable part adequately supported by guides disposed for said purpose underneath the fixed arm, in such a manner that said type of fork initially includes a transverse axle disposed by its ends in the holes of the brackets pertaining to the upper part of the fixed or longer arm so that, when actuated in a scissor-like manner, said mobile arm produces the upward or downward tilting thereof and therefore the displacement of the part disposed underneath and along the entire length of the fixed arm, said part having a toothed segment disposed transversely at its end, to which the teeth of the wheel pertaining to the needle-gripping head are geared, thereby achieving rotation of the head and producing the right and left movement, in accordance with the direction of rotation, of the needle so as to perform the corresponding suturing.

This second variant of the aforementioned embodiment involves neither pulleys nor tensioners, but rather the transmission is direct, in one case by means of the lateral plate that pushes the piston rod that closes the head and, in another, by means of the movable elongated part disposed underneath the fixed or longer arm, through which the toothed wheel that forms part of the head is rotated, thereby rotating said head, as in the preceding case, in one direction or other.

Although obvious to a person a skilled in the art, the described surgical needle holder is operated by introducing the ring finger through the scissor grip eye disposed at the end of the fixed arm, while the thumb is introduced through the scissor grip eye of the mobile arm, thereby actuating the needle holder or device in question in a scissor-like manner.

In the second variant of the described embodiment, the suture needle is gripped or held in a plane perpendicular to that in which the head is closed, i.e. in a different manner to that in which the needle is gripped or held in the needle holder corresponding to the first embodiment, wherein said needle is gripped in a longitudinal plane, the grip of the second version being more effective, although the functionality of both is optimal.

In another embodiment alternative, the lateral plate that comes into contact with the end of the axle associated to the head so as to open and close it, rather than being disposed on the opposite side of the head, may be disposed on the side of the head itself, acting directly on said head so as to close it, thereby eliminating the pushing piston rod and obviously the articulation means of said lateral plate against the fixed arm, although said lateral plate shall logically be articulated at a point in which its separation with respect to the main axle, originated by a force, in turn produces its separation from the end, pressing against the head so as to close it.

According to the aforementioned characteristics, it is possible to perform high-precision suturing in areas of the human body that are impossible to access using other devices or even by the surgeon's hand.

In the same manner, by virtue of the aforementioned characteristics of the needle holder, it is possible to rotate the needle-gripping head along a plane parallel to the needle holder's axle, allowing the needle to rotate in the same plane, and enabling the head to open and close by simply actuating a pawl in the first embodiment, and simply and partially rotating the mobile arm in the second embodiment, in addition to selecting the rotation direction of the head to enable effective and precise suturing, even allowing the needle to move at any angle and describe a circumference.

### DESCRIPTION OF THE DRAWINGS

For the purpose of complementing the preceding description and to further explain the characteristics of the invention, a set of drawings in accordance with a preferred embodiment thereof has been included as an integral part of said description, in which the following figures have been represented in an illustrative and unlimitative manner:
- Fig. 1.-: Shows a representation based on an exploded perspective view of the needle holder, executed in accordance with the object of the present invention, and showing all the constituent elements thereof.
- Fig. 2.-: Shows a longitudinal perspective view of the needle holder of the invention.
- Fig. 3.-: Shows a different longitudinal perspective view of the same needle holder represented in the preceding figure.
- Fig. 4.-: Shows in perspective view a detail of the needle-gripping clamp mounted laterally on the head of the needle holder.
- Fig. 5.-: Shows in perspective view a detail of the gear mechanism and selection of the direction of rotation which is transmitted to the head represented in the preceding figure.
- Fig. 6.-: Shows a representation based on a general perspective view of a second variant of embodiment of the needle holder of the invention.
- Fig. 7.-: Shows another perspective view, in this case of the other side, of the needle holder represented in the preceding figure.
- Fig. 8.-: Shows in perspective view a detail of the head assembly corresponding to the variant of embodiment of figures 6 and 7, in addition to the end of the movable sliding part disposed underneath the fixed arm, showing the gearing of the toothed wheel to the head on the rack segment formed at the end of said part.
- Fig. 9.-: Shows a detail of mobile arm actuation required to rotate the head, said actuation being carried out in a scissor-opening manner.
- Fig. 10.-: Finally shows in upper perspective view another detail of mobile arm rotation required to open and close the head.

### PREFERRED EMBODIMENT OF THE INVENTION

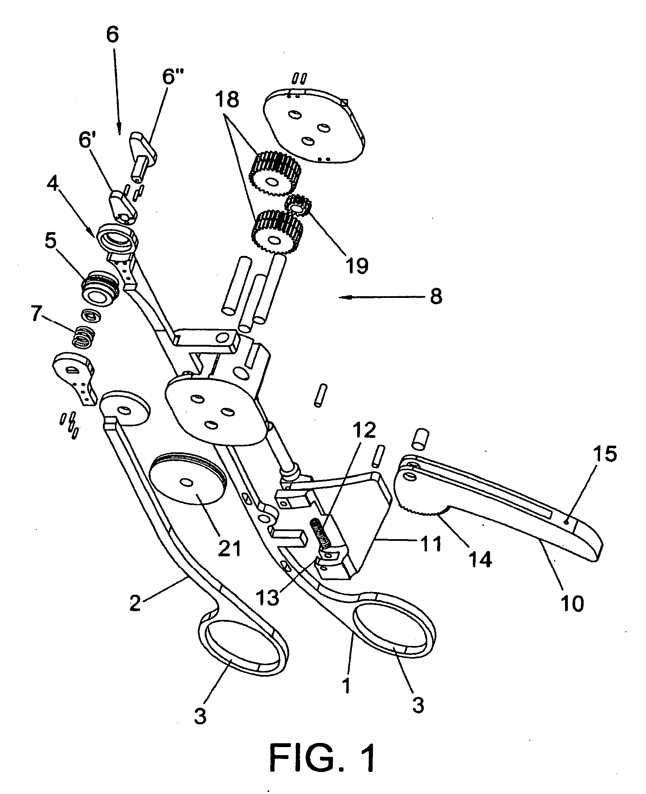

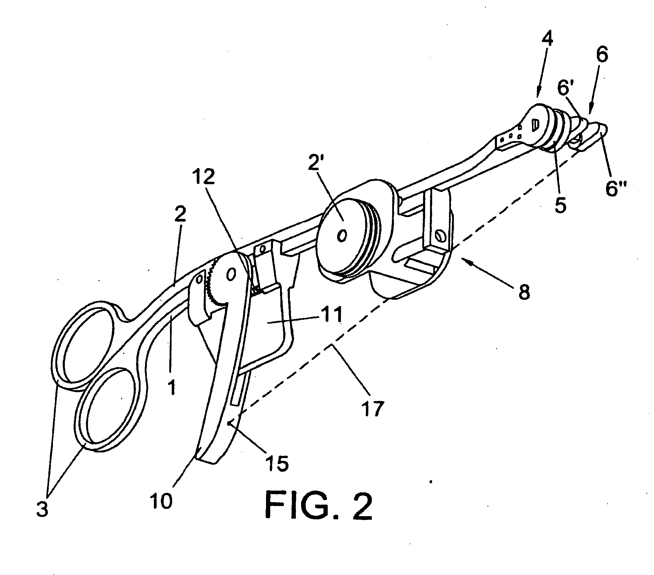

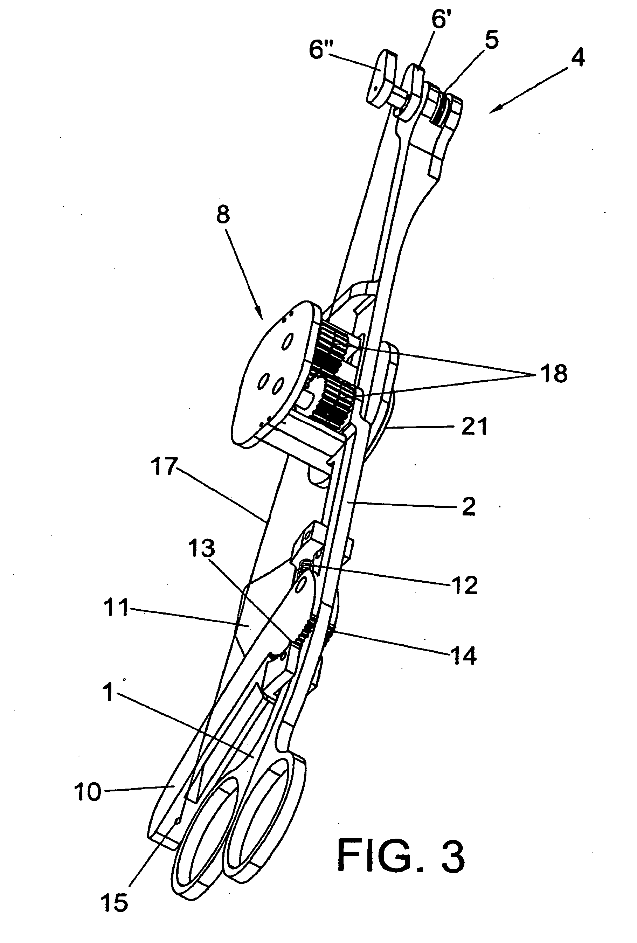

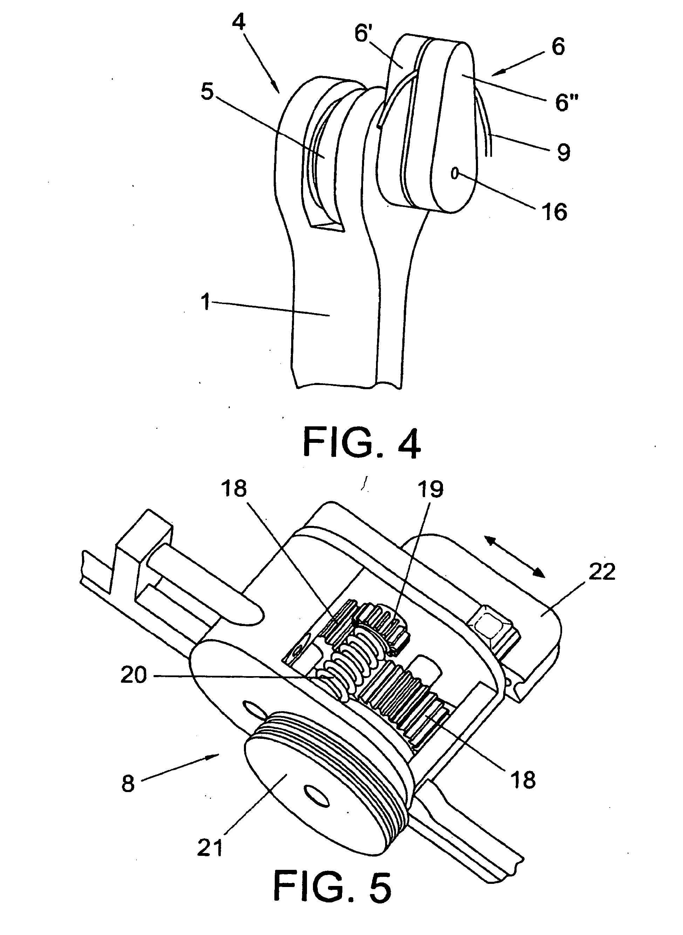

According to the preceding figures, and specifically in relation to figures 1 to 5 which show a preferred form of embodiment of the invention, we can see that the needle holder of the invention is comprised of two arms (1) and (2), the first being considerably longer than the second and both ending in expansions (3) by way of scissor grip eyes for fingers and by means of which to carry out manipulation or actuation, specifically of the mobile arm (2).

The fixed arm (1), as of the aforementioned mobile arm (2), has an extension at the end of which a head (4) is disposed, comprised of a pulley (5) and laterally, on one side, a clamp (6) with a fixed branch (6') and a mobile branch (6"), the latter being capable of movement in a forward and backward direction with respect to the fixed branch (6'), with the peculiarity that said clamp (6) is mounted on an extension of the pulley (5) axle, thereby complementing the head assembly with a spring (7).

The needle holder also includes a gear mechanism (8) which actuates the head, in such a manner that, by means of a belt or cable, not represented, and by means of a lateral belt (21) disposed in said mechanism (8), the movement is transmitted to the pulley (5) of the head (4), in order to produce rotation of the clamp (6).

In turn, said clamp (6) is capable of opening and closing for the lateral displacement of a mobile branch (6") that grips or releases the corresponding suture needle (9) which, as is already known, has a curved configuration.

The actuation of the mobile arm (2), and therefore transmission of movement, is only produced in a downward direction, i.e. in the direction in which the holder closes, in such a manner that the aforementioned clamp (6) is opened and closed by means of a pawl (10) mounted articulately on the fixed arm (1), said pawl (10) being associated to a ratchet (11) that moves axially on said arm (1), counter to a spring (12) that causes the teeth (13) of the ratchet (11) to come into permanent contact with a toothed segment (14) of the pawl (10), for the purpose of blocking said pawl in any actuation position.

A cable (17) passes between the pawl (10) and the mobile branch (6") of the clamp (6), fixed by one of its ends into a hole (16) and by the other end into a hole (16) of said mobile branch (6"), in such a manner that, when the pawl (10) is actuated, the clamp is closed and the needle (9) gripped so as to enable suturing, said closed position being blocked by means of the aforementioned ratchet (11), i.e. by the teeth thereof (13) which are geared to the toothed segment (14) of the pawl (10).

The removal or separation of the needle (9) shall be carried out by actuating the ratchet (11) and displacing it slightly along the fixed arm (1), counter to the spring (12), thereby releasing the toothed segment (14) and therefore the pawl (10) which, due to the tension of the cable (17), makes it tilt, detensioning the cable.

The direction of rotation of the clamp (6), and therefore the needle (9), so as to perform the suturing, is determined by the transmission of the rotation movement to the pulley (59 and therefore the clamp (6), said transmission being produced by the gear mechanism (8), which is mounted in correspondence with the mobile end (2) or, in other words, between the head 84) and the pawl (10) mounting area, in such a manner that said gear mechanism (8) comprises two toothed wheels (18), geared together, and therefore having opposite directions of rotation, although both wheels may rotate both in a clockwise and anti-clockwise direction, with the peculiarity that one of said wheels (18) is susceptible to having a pinion (19) selectively geared to it, due to which said pinion may be displaced with respect to its mounting axle so as to become geared to either wheel, thereby transmitting the rotation movement in either direction to the lateral and external pulley (21), between which and the head (4) pulley (5) the corresponding transmission belt shall be disposed.

This gear mechanism (8) is actuated by means of the mobile arm (2) in a scissor-like manner, i.e. by introducing the thumb through the scissor grip eye (3) which, when actuated, acts on one of the wheels (18) so as to produce the rotation that shall be transmitted to the pulley (21), as mentioned earlier, in such a manner that said rotation is transmitted from said pulley to the pulley (5), producing the rotation of the clamp (6) and therefore the adequate movement of the needle (9) for suturing.

The clockwise or anti-clockwise rotation of the pulley (5) is selected by displacing a mobile element (22) which acts as a selector.

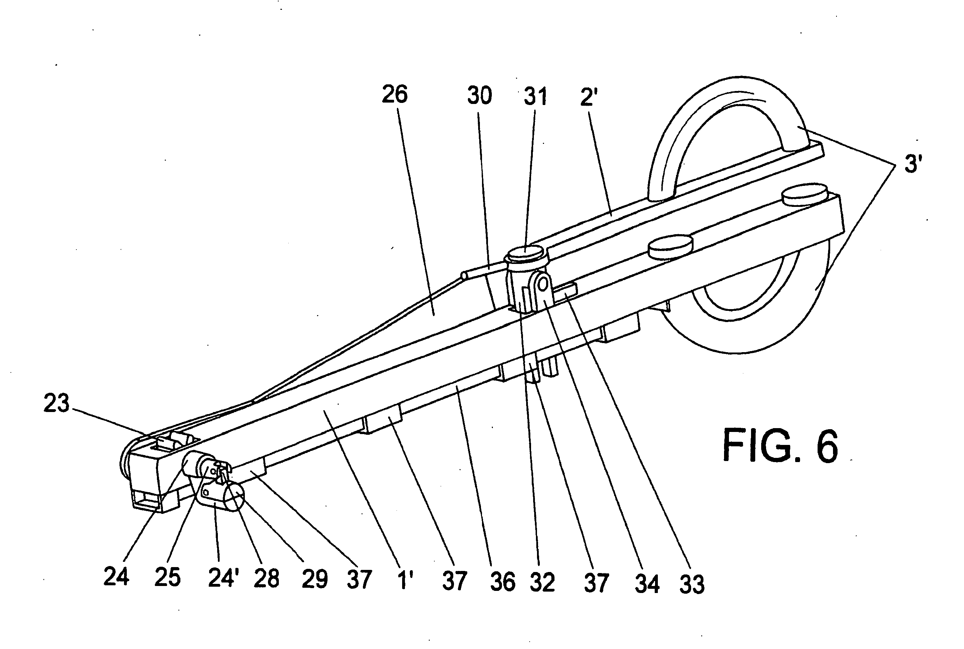

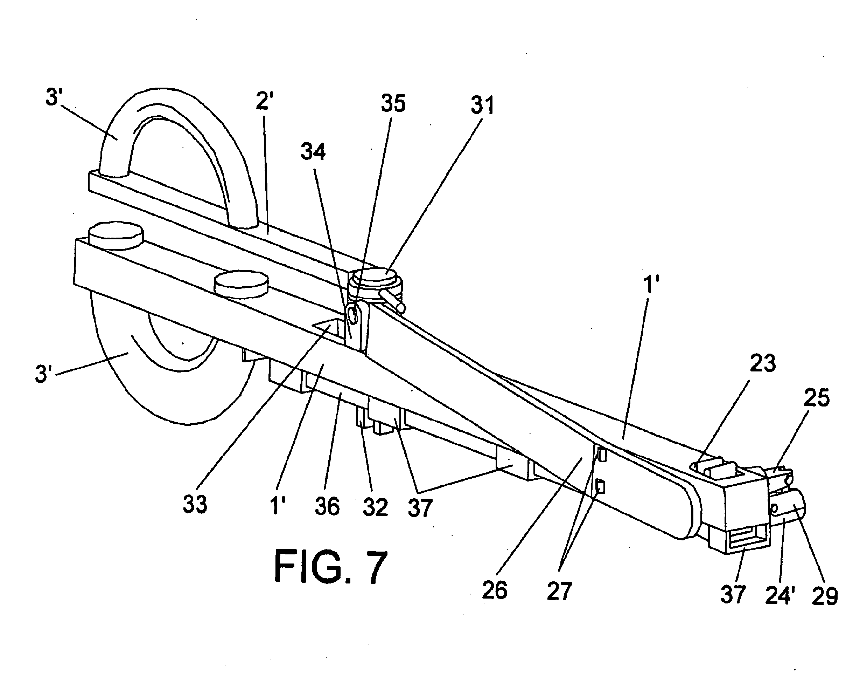

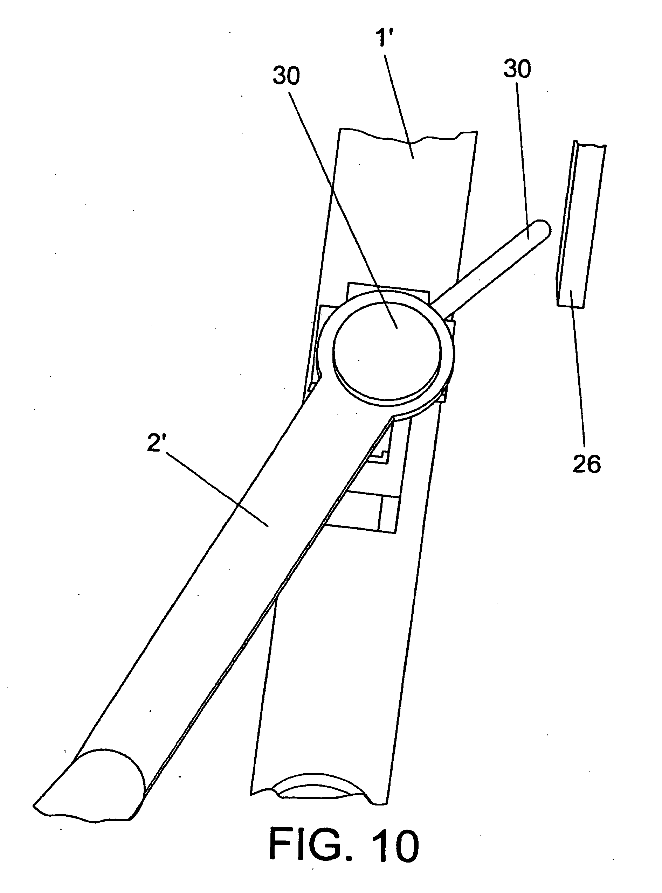

In an alternative embodiment shown in figures 6 to 10, the needle holder does not comprise pulleys or tensioners, wherein movement is transmitted directly, as described below.

In this case, the needle holder includes the fixed arm (1') and the mobile arm (2'), as mentioned earlier, with the scissor grip eye (3') being disposed at one of the ends of each of the arms, through which the fingers are introduced so as to actuate the needle holder.

In this variant of embodiment, the corresponding head (4') disposed at the end of the fixed arm (1') comprises a body (24) with a pinion (23) at one of the ends, in such a manner that an axle or piston rod (25) passes through the body (24) and the pinion (23) itself, said axle or piston rod (25) being capable of moving axially, in correspondence with a transverse direction with respect to the fixed arm (1') itself, wherein one of the ends of the axle or piston rod (25) is disposed facing a plate (26) that is mounted laterally with respect to the fixed arm (1') by means of tilting pivots (27) that project from the respective side of the fixed arm (1').

The other end of the piston rod or axle (25), disposed opposite to the end facing the lateral and tilting plate (26), is related to an appendix (28) pertaining to a tilting part (29) that tilts around a pass-through pin, the tilting movement of which is produced with respect to a fixed part (24') pertaining to the body (24) of the head (4) itself, in such a manner that the suture needle is disposed between said fixed part (24') and the tilting part (29), in a position that shall be established upon producing the tilting movement of the part (29), over its connection to the piston rod or axle (25) and its articulation over the aforementioned fixed part (24'), while if the plate (26) is pushed outwards laterally with respect to the fixed arm (1'), the section determined by the articulation formed by the pivots (27) produces the displacement to the side of the arm (1') itself, whereupon said plate (26) is supported and pushed against the end of the respective piston rod or axle (25), causing its displacement and therefore tilting movement towards the closed position of the part (29), which shall press against the fixed part (24') upon retaining and fixing the needle disposed between said two parts.

Said tilting movement of the lateral plate (26) to grip the suture needle is carried out by means of an appendix (30) disposed at the end of the mobile arm (2'), subsequent to the axle (31) with respect to which said mobile arm (2') may rotate. That is, when said mobile arm (2') is rotated with respect to the axle (31), said rotation produces the lateral displacement of the appendix (30) and therefore the outward pushing of the plate (26) with the opposite end thereof. That is, the part or section that extends from the articulation (27) of the plate (26) approaches the fixed arm (1'), thereby pushing the piston rod or axle (25), as mentioned earlier, with the previously described function.

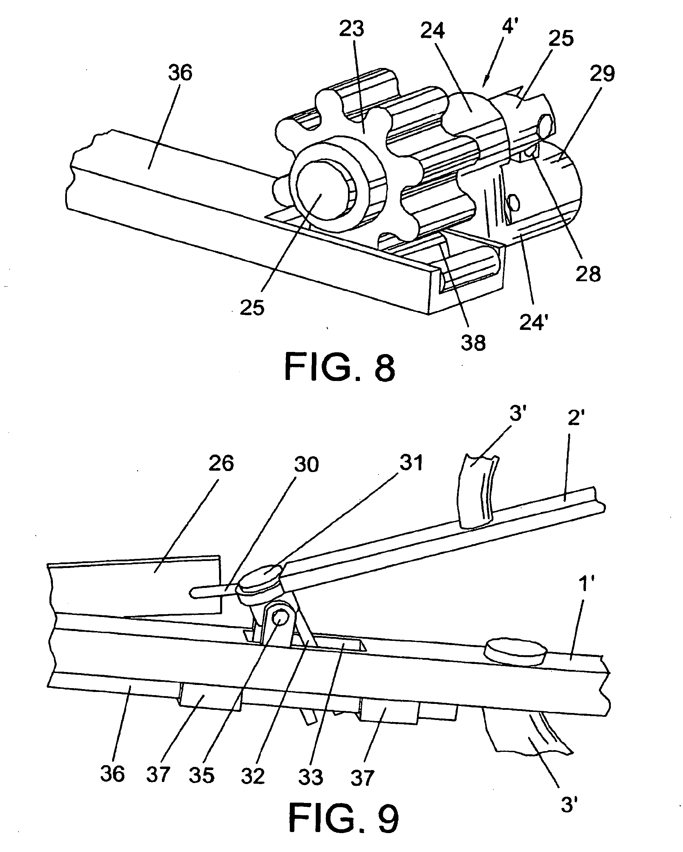

In turn, the aforementioned mobile arm (2') may tilt as represented in figure 9, which shows, underneath the lower part of the rotation axle (31) a part that divides into two branches that form a type of fork (32), which move and tilt inside a pass (33) disposed for said purpose in the fixed arm (1') itself, said type of fork (32) being articulated between brackets (34) disposed collaterally to the pass (33) and in correspondence with the upper part of the arm (1') itself, in such a manner that when the mobile arm (2') is actuated upwards as represented in figure 9, the tilting movement of the type of fork (32) is produced around the rotation axle (35) disposed between the brackets (34), said tilting movement causing the displacement of a part (36) disposed in a longitudinal manner and guided under the fixed arm (1), said guide being comprised of "U-shaped" profiles (37) disposed underneath the aforementioned fixed arm (1'), in such a manner that the displacement of said longitudinal part (36) is achieved upon the gearing of the type of fork (32) to a piston rod of said part (36), as a result of which the movement of the fork (32) produces the movement of said part (36). As a consequence of the movement of said part (36), the free end of which is provided with grooves in the form of a zip (38) to which the pinion (23) is geared, rotation is produced thereof, which in turn produces rotation of the body (24) that corresponds to the head (4') itself which, in accordance with the direction of the tilting movement and therefore of rotation of the pinion (23), rotation of the head (4') shall be produced in one direction or other, thereby actuating the suture needle gripped by said head so as to perform the corresponding suturing.

That is, in the variant of embodiment shown in figures 6 to 10, the mobile arm (2') may on one hand rotate around the axle (31) so as to open or close the head and therefore grip or release the suture needle and, on the other, said mobile arm (2') may tilt in such a manner as to produce rotation of the head and therefore of the suture needle, in either direction, so as to enable its functionality.

As mentioned earlier, the surgeon introduces his/her ring finger through the scissor grip eye (3) or (3') of the fixed arm (1) or (1'), while introducing the thumb through the scissor grip eye (3) or (3') of the mobile arm (2) or (2'), thereby actuating the needle holder in a scissor-like manner, in such a manner that the end of the fixed arm (1) or (1') is used to position the head in the part required by the surgeon.

Although no reference to the position of the pinion (23) is made in the description of the second form of embodiment, the figures show that it can be housed within a cavity disposed for said purpose next to the free end of the fixed arm (1'), a lateral hole being disposed in correspondence with said cavity through which a part of the body (24) corresponding to the head (4') passes and is positioned, also showing that, with the exception of said pinion (23), the rest of the head projects through one of the sides of the fixed arm (1'), even allowing the lateral pushing part or plate (26) of the piston rod or axle (25) to be disposed on said side.

With regard to the tilting movement of the mobile arm (2') required to produce displacement of the internal part (36) and therefore rotation of the head (4'), we must comment that a maximum tilting movement shall produce a maximum displacement of said part, in such a manner that the tactile sensation reached by said maximum is achieved by means of a deformable and elastic part, not represented, which can be a spring, elastic sheet or similar, which generates a progressive resistance in the axle or fork (32), which informs the manipulator, i.e. the surgeon, that the part (32) is ungeared with respect to the pinion (23) and that, therefore, the head (4') moves freely at that point. Specifically, said deformable part is in contact with the end of the part (36) by the scissor grip eyes of the arm (1') and, when disposed opposite to the head upon moving, the zip (38) comes into contact with the end of the part (36), in such a manner that the part or body (24) may move freely. In this manner, the head may be disposed according to the "starting angle" that best adapts to the intervention, providing an additional 140° of geared movement, corresponding to the maximum longitudinal displacement that the tilting movement of the fork (32) is capable of transmitting to the part (36), which in turn is transformed into the angular movement of the body (24) by means of the pinion (23).

## Claims

1. Surgical needle holder for stitch suturing which, being configured to facilitate stitch suturing in areas that are difficult or impossible to access by the surgeon's hand, in addition to enabling high-precision suturing, particularly in those cases in which the plane of the needle corresponds to the longitudinal plane of the needle holder, comprising two arms (1) or (1') and (2) or (2'), the first being fixed and longer than the mobile arm (2) or (2') with the peculiarity that both the fixed arm (1) or (1') and the mobile arm (2) or (2') include, at one of their ends, a scissor grip eye (3) or (3') through which the manipulator or surgeon introduces his/her fingers, thereby actuating the needle holder itself in a scissor-like manner **characterized in that** it includes the free on end of the fixed arm (1) or (1') a head (4) or (4') capable of rotating in one direction or other so as to enable the corresponding suture needle gripped by said head to perform the corresponding suturing, said head (4) or (4') having means to hold the needle in place or release it; also including means that enable actuation of the mobile arm (2) or (2') so as to close or open the head and therefore grip or release the suture needle or rotate said head (4) or (4') to enable the suture needle to perform its function.

2. Surgical needle holder for stitch suturing, according to claim 1, **characterized in that** the head (4) comprises a clamp (6) with a fixed branch (6') and another mobile branch (6"), having an axle capable of producing axial movement or rotation, on which a pulley (5) is in turn mounted, on a free and lateral end thereof, by means of which the rotation movement is transmitted to said clamp (6) of the head (4), said pulley (5) being related to a second driving pulley (21), through the corresponding belt or chain, mounted on a gear mechanism (8).

3. Surgical needle holder for stitch suturing, according to the preceding claims, **characterized in that** the gear mechanism (8) comprises two toothed wheels (18), geared together with opposite directions of rotation, said direction of rotation being selected by means of a laterally disposed mobile element (22), being susceptible to gearing with either of said wheels (18) and, selectively, a pinion (19) that transmits the rotation movement to the driving pulley (21) and, therefore, by means of the corresponding belt, to the head (4) pulley (5), and from there to the clamp (6) that comprises the gripping element of the suture needle (9).

4. Surgical needle holder for stitch suturing, according to claim 1, **characterized in that** the lateral displacement of the mobile branch (6") of the clamp (6), required to release or grip the needle (9), is carried out by means of a manually actuated pawl (10) related to a ratchet (11) that blocks said pawl (10) and is geared to a toothed segment (14) of the pawl (10) itself, counter to a spring (12) that tends to constantly produce gearing of the teeth (13) to the toothed segment (14), and therefore blocking of the pawl (10), so as to maintain the tension of a cable (17) fixed by its ends between the pawl (10) itself and the mobile branch (6") of the clamp (6).

5. Surgical needle holder for stitch suturing, according to claims 1 and 4, **characterized in that** the ratchet (11) is movably mounted on the fixed arm (1) so as to unblock the teeth (13) of the ratchet (11) from the toothed segment (14) of the pawl (10).

6. Surgical needle holder for stitch suturing, according to claim 1, **characterized in that** the head (4') consists of a body (24) comprised of a pinion (23) housed in a cavity disposed for said purpose at the free end of the fixed arm (1'), which laterally projects from the body (24) of the head (4'), wherein a pass-through pin or axle (25) passes transversely through both the pinion (23) and the body (24) of said head, the end of which is disposed facing the end of a lateral plate (26) that is articulately mounted on the fixed arm (1'), said plate (26) being actuated by the rotation of the mobile arm (2'), in such a manner that said tilting movement causes it to press against the end of the axle or piston rod (25) and move towards the opposite side, coming into contact with an appendix of a mobile part (29) that opens and closes, and is articulately mounted with respect to a fixed part (24') of the head itself.

7. Surgical needle holder for stitch suturing, according to claims 1 and 6, **characterized in that** the mobile arm (2') is mounted on an axle (31) in such a manner that an appendix (30) of said mobile arm (2') comes into contact with the fore-end of the lateral plate (26) and causes it to tilt, thereby pushing the opening and closing piston rod or axle (25) of the head that grips the suture needle, under the axle of which a fork-like body (32) is disposed in a pass (33) of the fixed arm (1'), said fork-like body (32) being mounted in a tilting manner between two brackets (34), in such a manner that, when actuated, the mobile arm (2') produces the forward or backward tilting of said fork (32) and therefore the displacement of a longitudinal and lower part (36) disposed underneath the fixed arm (1') and between profiles (37), said part (36) having a zip (38) disposed at its free end, to which the pinion (23) is geared in such a manner that the displacement thereof produces the rotation of said pinion (23) and therefore rotation of the head (4'), so as to enable the corresponding functionality of the suture needle.

## Patentansprüche

1. Ein chirurgischer Nadelhalter für das chirurgische Nähen, wobei dieser dazu konfiguriert ist, ein Nähen in Bereichen zu erleichtern, die für die Hand des Chirurgen schwer oder nicht zugänglich sind, wobei zusätzlich ein Präzisionsnähen ermöglicht wird, vor allem in den Fällen, in denen die Ebene der Nadel der Längsebene des Nadelhalters entspricht, wobei dieser zwei Arme (1) oder (1') und (2) oder (2') umfasst, wobei der erste feststehend und länger als der bewegliche Arm (2) oder (2') ist, mit der Besonderheit, dass sowohl der feststehende Arm (1) oder (1') und der bewegliche Arm (2) oder (2') an einem seiner Enden ein Scherengriffauge (3) oder (3') einschließen, durch welches der Bediener oder Chirurg seine/ihre Finger einführt, wodurch der Nadelhalter selbst in einer scherenartigen Weise betätigt wird, **dadurch gekennzeichnet, dass** dieser auf dem freien Ende des feststehenden Arms (1) oder (1') einen Kopf (4) oder (4') einschließt, der dazu in der Lage ist, sich in die eine oder andere Richtung zu drehen, um es der entsprechenden, von dem besagten Kopf gehaltenen Suturnadel zu ermöglichen, die Nadel an der Stelle zu halten oder diese freizugeben; wobei dieser ebenfalls Mittel einschließt, welche die Betätigung des beweglichen Arms (2) oder (2') ermöglichen, um den Kopf zu schließen oder zu öffnen und folglich die Suturnadel zu greifen oder freizugeben, oder um den besagten Kopf (4) oder (4') zu drehen, damit es der Suturnadel ermöglichen wird, deren Funktion auszuführen.

2. Ein chirurgischer Nadelhalter für das chirurgische Nähen nach Anspruch 1, **dadurch gekennzeichnet, dass** der Kopf (4) eine Klammer (6) mit einem feststehenden Schenkel (6') und einem anderen beweglichen Schenkel (6") umfasst, wobei diese eine Achse besitzt, die dazu in der Lage ist, eine axiale Bewegung oder Rotation zu erzeugen, auf der an deren freiem und seitlichem Ende wiederum eine Rolle (5) montiert ist, über welche die Rotationsbewegung an die besagte Klammer (6) des Kopfes (4) übertragen wird, wobei die besagte Rolle (5) über einen entsprechenden Riemen oder eine Kette mit einer zweiten Antriebsrolle (21) in Beziehung steht, die auf einem Zahnradgetriebe (8) montiert ist.

3. Ein chirurgischer Nadelhalter für das chirurgische Nähen nach den vorherigen Ansprüchen, **dadurch gekennzeichnet, dass** das Zahnradgetriebe (8) zwei Zahnräder (18), die mit entgegengesetzter Drehrichtung miteinander verzahnt sind, wobei die besagte Drehrichtung mittels eines seitlich angeordneten beweglichen Elements (22) ausgewählt wird, das dafür empfänglich ist, in eines der besagten Räder (18) zu greifen, und wahlweise ein Ritzel (19) umfasst, das die Drehbewegung an die Antriebsrolle (21) und somit mittels des entsprechenden Riemens an die Kopf- (4) Rolle (5) und von da aus an die Klammer (6) überträgt, welche das Greifelement der Suturnadel (9) umfasst.

4. Ein chirurgischer Nadelhalter für das chirurgische Nähen nach Anspruch 1, **dadurch gekennzeichnet, dass** die seitliche Verschiebung des beweglichen Schenkels (6") der Klammer (6), welche zur Freigabe oder Ergreifung der Nadel (9) erforderlich ist, mittels einer manuell betätigten Sperrklinke (10) durchgeführt wird, die mit einer Sperrvorrichtung (11) in Beziehung steht, welche die besagte Sperrklinke (10) blockiert und mit einem gezahnten Segment (14) der Sperrklinke (10) selbst verzahnt ist, einer Feder (12) entgegenwirkend, die dazu neigt, eine ständige Verzahnung der Zähne (13) mit dem gezahnten Segment (14) zu erzeugen und somit die Sperrklinke (10) zu blockieren, um die Spannung des mittels seiner Enden zwischen der Sperrklinke (10) selbst und dem beweglichen Schenkel (6") der Klammer (6) befestigten Kabels (17) aufrechtzuerhalten.

5. Ein chirurgischer Nadelhalter für das chirurgische Nähen nach Anspruch 1 und 4, **dadurch gekennzeichnet, dass** die Sperrvorrichtung (11) beweglich auf dem feststehenden Arm (1) montiert ist, um die Zähne (13) der Sperrvorrichtung (11) von dem gezahnten Segment (14) der Sperrklinke (10) zu entsperren.

6. Ein chirurgischer Nadelhalter für das chirurgische Nähen nach Anspruch 1, **dadurch gekennzeichnet, dass** der Kopf (4') aus einem Gehäuse (24) gebildet wird, das aus einem Ritzel (23) besteht, welches in einem Hohlraum aufgenommen ist, der zu diesem Zweck an dem freien Ende des feststehenden Arms (1') vorgesehen ist, der seitlich aus dem Gehäuse (24) des Kopfes (4) herausragt, in dem ein Passierstift oder eine Achse (25) quer sowohl durch das Ritzel (23) als auch das Gehäuse (24) des besagten Kopfes verläuft, dessen Ende so angeordnet ist, dass dieses dem Ende der seitlichen Platte (26) zugewandt ist, die gelenkig auf dem feststehenden Arm (1') montiert ist, wobei die besagte Platte (26) durch die Rotation des beweglichen Arms (2') auf eine solche Weise betätigt wird, dass die besagte Kippbewegung diese dazu veranlasst, gegen das Ende der Achs- oder Kolbenstange (25) zu drücken und sich zu der entgegengesetzten Seite zu bewegen, wobei diese in Kontakt mit einem Anhang des beweglichen Teils (29) kommt, das sich öffnet und schließt und gelenkig gegenüber einem feststehenden Teil (24') des Kopfes selbst montiert ist.

7. Ein chirurgischer Nadelhalter für das chirurgische Nähen nach Anspruch 1 und 6, **dadurch gekennzeichnet, dass** der bewegliche Arm (2') auf eine solche Weise auf einer Achse (31) montiert ist, dass ein Anhang (30) des besagten beweglichen Arms (2') in Kontakt mit dem Vorderende der seitlichen Platte (26) kommt und diese dazu bringt, sich zu neigen, wodurch die Öffnungs- und Schließ-Achsstange oder Kolbenstange (25) des Kopfes, welche die Suturnadel greift, gedrückt wird, unter deren Achse ein gabelartiges Gehäuse (32) in einem Durchgang (33) des feststehenden Arms (1') angeordnet ist, wobei das besagte gabelartige Gehäuse (32) auf eine gekippte Weise derartig zwischen zwei Bügeln (34) montiert ist, dass der bewegliche Arm (2') bei Betätigung desselben die Vorwärts- oder Rückwärtsneigung der besagten Gabel (32) erzeugt und somit die Verschiebung eines längslaufenden und unteren Teils (36), das unter dem feststehenden Arm (1') und zwischen Profilen (37) angeordnet ist, wobei das besagte Teil (36) einen Reißverschluss (38) besitzt, der an dessen freiem Ende angeordnet ist, mit dem das Ritzel (23) auf eine solche Weise verzahnt ist, dass die Verschiebung desselben die Rotation des besagten Ritzels (23) und somit die Rotation des Kopfes (4') erzeugt, um die entsprechende Funktionalität der Suturnadel zu ermöglichen.

## Revendications

1. Porte aiguille chirurgicale pour la suture de point, conçu pour faciliter la suture de point à des endroits difficilement accessibles ou inaccessibles aux mains du chirurgien, permettant en outre une réalisation de points de suture extrêmement précise, en particulier dans les zones dans lesquelles le plan de l'aiguille correspond au plan longitudinal du porte aiguille, équipé de deux bras (1) ou (1') et (2) ou (2'), le premier bras étant fixe et d'une longueur plus importante que le bras mobile (2) ou (2') avec la particularité de ce qu'à la fois le bras fixe (1) ou (1') et le bras mobile (2) ou (2') comprennent, au niveau de l'une de leurs extrémité, des ciseaux dotés d'un anneau de préhension de (3) ou (3') à travers lequel le manipulateur ou le chirurgien introduit un ou plusieurs doigts, le porte aiguille lui-même faisant office de ciseau , **caractérisé en ce qu'**il comprend au niveau de l'extrémité mobile du bras fixe (1) ou (1'), une tête (4) ou (4') capable de pivoter dans un sens ou un autre de sorte à permettre à ladite tête de saisir l'aiguille de suture correspondante afin qu'elle réalise le point de suture correspondant, ladite tête (4) ou (4') étant dotée de moyens pour maintenir l'aiguille en place ou pour la libérer ; comprenant également des moyens permettant le mouvement du bras mobile (2) ou (2') de sorte à permettre la fermeture ou l'ouverture de la tête et de ce fait saisir ou libérer l'aiguille de suture ou faire pivoter ladite tête (4) ou (4') afin de permettre à l'aiguille de suture de réaliser sa fonction.

2. Porte aiguille chirurgicale pour la suture de point, selon la revendication 1, **caractérisé en ce que** la tête (4) comprend une pince (6) dotée d'une branche fixe (6') et une autre branche mobile (6"), équipée d'un axe capable de produire un mouvement axial ou de rotation, sur lequel une poulie (5) a été montée, sur une extrémité libre et latérale, au moyen de laquelle le mouvement rotatif est transmis sur ladite pince (6) de la tête (4), ladite poulie (5) étant reliée à une deuxième poulie de guidage (21), à travers la courroie ou chaine correspondante, montée sur un système d'engrenage (8).

3. Porte aiguille chirurgicale pour la suture de point, selon les revendications précédentes, **caractérisé en ce que** le système d'engrenage (8) comporte deux roues dentées (18), tournant dans des sens de rotation opposés, ledit sens de rotation étant sélectionné au moyen d'un élément mobile (22) placé latéralement, à même de faire pivoter n'importe laquelle desdites roues (18) ou bien un pignon (19) qui transmettra le mouvement de rotation à la poulie de guidage (21) et, par conséquent, au moyen de la courroie correspondante, à la poulie (5) de la tête (4), et donc à la pince (6) qui comporte l'élément de préhension de l'aiguille de suture (9).

4. Porte aiguille chirurgicale pour la suture de point, selon la revendication 1, **caractérisé en ce que** le déplacement latéral de la branche mobile (6") de la pince (6), nécessaire à la libération ou à la saisie de l'aiguille (9), s'effectue au moyen d'un cliquet (10) actionné à la main et relié à un rochet (11) destiné à immobiliser ledit cliquet (10) et qui fait pivoter un segment denté (14) du cliquet (10) lui-même, à l'encontre d'un ressort (12), qui tend à produire l'embrayage permanent des dents (13) sur le segment denté (14), et de ce fait l'immobilisation du cliquet (10), de sorte à conserver la tension d'un câble (17) dont les extrémités sont reliées entre le cliquet (10) lui-même et la branche mobile (6") de la pince (6).

5. Porte aiguille chirurgicale pour la suture de point, selon les revendications 1 et 4, **caractérisé en ce que** le rochet (11) est monté de manière amovible sur le bras fixe (1) de sorte à libérer les dents (13) du rochet (11) du segment denté (14) du cliquet (10).

6. Porte aiguille chirurgicale pour la suture de point, selon la revendication 1, **caractérisé en ce que** la tête (4') est constituée d'un corps (24) comprenant un pignon (23) placé dans un orifice réalisé à cette fin au niveau de l'extrémité libre d'un bras fixe (1') qui s'étend latéralement à partir du corps (24) de la tête (4'), dans lequel une broche ou axe (25) est introduite transversalement à travers le pignon (23) et le corps (24) de ladite tête, dont l'extrémité est placée face à l'extrémité d'un plateau latéral (26) articulé monté sur le bras fixe (1'), ledit plateau (26) étant actionné par la rotation du bras mobile (2'), de sorte à ce que ledit mouvement de basculement provoque une pression sur l'extrémité de l'axe ou tige de piston (25) et le dirige vers le côté opposé, pour entrer en contact avec une partie annexe de la partie mobile (29) pouvant s'ouvrir et se fermer, et articulée sur une partie fixe (24') de la tête elle-même.

7. Porte aiguille chirurgicale pour la suture de point, selon les revendications 1 et 6, **caractérisé en ce que** le bras mobile (2') est monté sur un axe (31) de telle sorte que la partie annexe (30) dudit bras mobile (2') entre en contact avec la partie frontale du plateau latéral (26) pour le faire entrer en mouvement, et de ce fait pousser l'axe ou tige de piston (25) de la tête de préhension de l'aiguille de suture destiné à l'ouverture et à la fermeture, en dessous de l'axe où un corps en forme de fourche (32) a été placé dans un passage (33) du bras fixe (1'), ledit corps en forme de fourche (32) étant monté de sorte à produire un mouvement de basculement entre deux crochets (34), de sorte à ce que, lorsqu'il est mis en mouvement, le bras mobile (2') fasse basculer en avant et en arrière ladite fourche (32) et qu'il entraine par conséquent le déplacement d'un élément inférieur longitudinal (36) placé en dessous du bras fixe (1') et entre les sections (37), ledit élément (36) étant doté d'une fermeture à glissière (38) placée au niveau de son extrémité libre, que le pignon (23) fait pivoter de sorte à ce que son déplacement entraine la rotation dudit pignon (23) et par conséquent la rotation de la tête (4'), de sorte à permettre le fonctionnement adéquat de l'aiguille de suture.
